# EUROPEAN PATENT APPLICATION

(11) **EP 1 782 827 A1**
(43) Date of publication of application: **09.05.2007**
(21) Application number: 05752965.3
(22) Date of filing: 21.06.2005
(51) Int. Cl.: A61K 39/00, A61P 35/00

(54) **PEPTIDE VACCINE FOR CANCER THERAPY**

(30) Priority: 21.06.2004 JP 2004182811
(71) Applicant: Green Peptide Co., Ltd., Kurume-shi, Fukuoka 8300018 (JP)
(72) Inventor: ITOH, Kyogo, Miyaki-gun, Saga 8410205 (JP)
(74) Representative: Vossius & Partner
(86) International application number: PCT/JP2005/011357
(87) International publication number: WO 2005/123122

(57) **Abstract**

The present invention provides a method for treating a cancer patient which comprises administering a therapeutically effective amount of a cancer antigen peptide to the patient in need thereof, wherein the method comprises the steps of
1) providing a set of cancer antigen peptides comprising two or more cancer antigen peptides,
2) determining whether or not the patient has a peptide-specific cytotoxic T lymphocyte precursor which is specific to each peptide in the set of cancer antigen peptides,
3) removing the cancer antigen peptide to which the patient does not have the peptide-specific cytotoxic T lymphocyte precursor from the set of cancer antigen peptides to provide an administration set of cancer antigen peptides,
4) administering the administration set of cancer antigen peptides to the patient.

## Description

### TECHNICAL FIELD

The invention relates to a novel therapeutic method for the treatment of cancers.

### BACKGROUND OF THE INVENTION

We previously reported a series of antigenic peptides capable of inducing tumor-reactive cytotoxic T lymphocytes (CTLs) in HLA-A24⁺ or HLA-A2⁺ patients (1-5). Subsequently, we conducted clinical trials of peptide vaccination using two different regimens (6-12). In the first regimen, lung, colon, and cervical cancer patients were vaccinated with CypB-, SART3-, and SART2-derived peptides, respectively (6, 7, 12). Although the vaccination protocols were completely safe, neither the objective clinical effect nor the immune responses were observed. In the second regimen, named as "personalized" vaccination, HLA-A24⁺ or HLA-A2⁺ patients with advanced epithelial cancer were vaccinated with peptides for which the existence of CTL precursors was confirmed in the pre-vaccination peripheral blood mononuclear cells (PBMCs). Increased cellular and humoral immune responses to the vaccinated peptides were observed at higher frequencies in the post-vaccination PBMCs and sera, respectively (8-12). Furthermore, three of five patients with uterine cervical cancer showed objective tumor regression (12), and two of four patients with scirrhous gastric cancer (GC) have experienced progression-free conditions for more than 2 years (11).

### DISCLOSURE OF THE INVENTION

### PROBLEM TO BE SOLVED BY THE INVENTION

An object of the invention is to provide a method of peptide vaccination which is safe and therapeutically effective for cancer patients.

### MEANS FOR SOLVING THE PROBLEM

The inventors performed a new trial of a "pre-designated" regimen in which cancer patients were vaccinated with peptides which were frequently selected as vaccine candidates in the preceding personalized vaccine regimen (8-12). Eighteen cancer patients (10 with uterine cervical cancer and 8 with gastric cancer) were enrolled in a pre-designated regimen. The pre-designated regimen was well tolerated by all patients. Although peptide-specific CTL precursors and humoral immune responses increased in the majority of patients with the pre-designated regimen, neither of both responses correlated with clinical outcome.
Three patients had long stable disease, and their pre-vaccination peripheral blood mononuclear cells contained peptide-specific CTL precursors reactive to more than 2 of 4 peptides. With the pre-designated regimen, the levels of pre-existing immunoglobulin Gs reactive to non-vaccinated peptides decreased in 5 of 15 patients with progressive disease, and their time to progression were very short, whereas such a decrease was rarely observed in the preceding personalized regimen. These results suggest that the pre-designated regimen can elicit a primary immune response, but may incidentally suppress pre-existing immune responses when the peptide not recognized by the immune system of the patient is included in the vaccinated peptides.

One of the goals of tumor immunology is to develop a safe and therapeutically effective regimen of peptide vaccination for advanced cancer patients. However, no such regimen is available at the present time, despite the fact that many clinical trials of peptide vaccination regimens have been conducted in the past decade (6-12, 15-21). To achieve this goal, we conducted this study to evaluate the safety, immunological and clinical responses of the pre-designated peptide vaccination of four peptides for which CTL precursors were found in pre-vaccination PBMCs at higher frequencies and were administered to patients as vaccines in the preceding regimen of the personalized vaccination (8-12). As a result, cellular and humoral immune responses to the vaccinated peptides were effectively induced. The trial of the pre-designated regimen was essentially a prospective study of the previous personalized regime. In the pre-designated regimen, the levels of peptide-specific CTL precursors in the pre-vaccination PBMCs seemed to correlate with clinical outcome regarding both time to progression (TTP) and median survival time (MST). In contrast, although the levels of either peptide-induced cellular or humoral immune responses were strongly induced in the post-vaccination PBMCs or sera of the majority of patients tested, they did not correlate with clinical outcome. Based on these results, the inventors have accomplished the present invention, in which a set of cancer antigen peptides prepared by removing a cancer antigen peptide not recognized by the immune system of the patient from a set of candidate cancer antigen peptides is administered to the patient.

The present invention is based on the findings as shown above.
Thus, the invention provides a method for treating a cancer patient which comprises administering a therapeutically effective amount of a cancer antigen peptide to the patient in need thereof, wherein the method comprises the steps of
1) providing a set of cancer antigen peptides comprising two or more cancer antigen peptides,
2) determining whether or not the patient has a peptide-specific cytotoxic T lymphocyte precursor which is specific to each peptide in the set of cancer antigen peptides,
3) removing the cancer antigen peptide to which the patient does not have the peptide-specific cytotoxic T lymphocyte precursor from the set of cancer antigen peptides to provide an administration set of cancer antigen peptides,
4) administering the administration set of cancer antigen peptides to the patient.

The present invention further provides a kit for treating a cancer patient, which comprises
a set of cancer antigen peptides comprising two or more cancer antigen peptides, and
a reagent for determining whether or not the cancer patient has a peptide-specific cytotoxic T lymphocyte precursor which is specific to a cancer antigen peptide.

### BRIEF DESCRIPTION OF THE DRAWINGS

Figure 1 represents graphs showing the cellular responses to the vaccinated peptides in four representative cases of cervical cancer (C-1, 7, 9, and 10) and 2 GC cases (GC-4 and 8).
Figure 2 represents graphs showing that the serum IgGs reactive to the vaccinated peptides.
Figure 3 represents a graph showing the survival time of the patients under the two vaccination regimens for cervical cancer and sccirhous type GC patients.
Figure 4 represents graphs showing the serum IgGs reactive to non-vaccinated peptides.

### DETAILED DESCRIPTION OF THE PREFERRED EMBODIMENTS

In the first aspect, the present invention provides a method for treating a cancer patient which comprises administering a therapeutically effective amount of a cancer antigen peptide to the patient in need thereof, wherein the method comprises the steps of
1) providing a set of cancer antigen peptides comprising two or more cancer antigen peptides,
2) determining whether or not the patient has a peptide-specific cytotoxic T lymphocyte precursor which is specific to each peptide in the set of cancer antigen peptides,
3) removing the cancer antigen peptide to which the patient does not have the peptide-specific cytotoxic T lymphocyte precursor from the set of cancer antigen peptides to provide an administration set of cancer antigen peptides,
4) administering the administration set of cancer antigen peptides to the patient.

The term "cancer antigen peptide" as used herein means a tumor antigen peptides derived from tumor antigen proteins. Tumor antigen peptides are generated by degradation of tumor antigen proteins, which are proteins specific for tumors, in proteasomes of cells, in other words, are intracellularly synthesized. The tumor antigen peptides thus generated bind to MHC class I antigens (HLA antigens) in endoplasmic reticulum to form complexes, and the complexes are transported to the cell surface to be presented as an antigen.

Tumor antigen proteins as used herein include a protein named MAGE from human melanoma cells (Science, 254:1643, 1991); melanosomal proteins such as a melanocytic tissue-specific protein, gp100 (J. Exp. Med., 179:1005, 1994), MART-1 (Proc. Natl. Acad. Sci. USA, 91:3515, 1994), and tyrosinase (J. Exp. Med., 178:489, 1993); MEGE-related proteins (J. Exp. Med., 179:921, 1994); β-catenin having a tumor-specific amino acid mutation (J. Exp. Med., 183:1185, 1996); and CDK4 (Science, 269:1281, 1995); HER2-neu (J. Exp. Med., 181:2109, 1995), p53 (variant) (Proc. Natl. Acad. Sci. USA, 93:14704, 1996); tumor markers such as CEA (J. Natl. Cancer Inst., 87:982, 1995), PSA (J. Natl. Cancer Inst., 89:293, 1997); and viral proteins such as HPV (J. Immunol., 154:5934, 1995) and EBV (Int. Immunol., 7:653, 1995). Detailed descriptions of these substances can be found in published reviews (e.g. Immunol. Today, 18:267, 1997; J. Exp. Med., 183:725, 1996; and Curr. Opin. Immunol., 8:628, 1996).

Typical examples of tumor antigen peptides as used herein include, but not limited to, tumor antigen peptides described in WO97/46676, WO99/29715 and WO99/33977; tumor antigen peptides derived from cyclophilin B (WO99/67288); tumor antigen peptides derived from SART-1 (WO00/06595); tumor antigen peptides derived from SART-3 (WO00/12701); tumor antigen peptides derived from ART-1 (WO00/32770); tumor antigen peptides derived from SART2 (J. Immunol., 164:2565, 2000); tumor antigen peptides derived from lck (Eur. J. Immunol., 31:323, 2001); tumor antigen peptides derived from ART4 (Cancer Res., 60:3550, 2000); and tumor antigen peptides derived from ppMAPkk, WHSC2, UBE2V, HNRPL, EIF (Cancer Res., 61:2038, 2001).

Cancer antigen peptides as used in the invention can be identified by synthesizing candidate peptides which comprise a part of a tumor antigen protein, and screening for their ability to induce peptide-specific cytotoxic T lymphocytes (CTLs), and if necessary, additionally for their ability to be recognized by immunoglobulin Gs (IgGs). Synthesis of peptides may be conducted according to a method usually used in peptide chemistry. Examples of the known methods are those described in the literatures including "Peptide Synthesis", Interscience, New York, 1966; "The Proteins", vol. 2, Academic Press Inc., New York, 1976; "Pepuchido-Gosei", Maruzen Co. Ltd., 1975. Preferably, the peptides are synthesized based on the HLA binding motif. Screening of peptide candidates for their ability to induce peptide-specific CTLs usually may be conducted by an assay for determining whether or not the candidate peptide presented by an HLA antigen is recognized by CTL. Screening of peptide candidates for their ability to be recognized by IgGs usually may be conducted by ELISA.

Those skilled in the art could readily prepare and use "derivative of a cancer antigen peptide having the functionally equivalent properties thereof" which is an altered peptide whose amino acid sequence contains substitution, deletion and/or addition of one to several amino acid residues in the amino acid sequence of the peptide of the present invention, and which have the properties as a cancer antigen peptide, i.e., is to be capable of binding to an HLA antigen and being recognized by CTL, and if any to be recognized by humoral immune response. Cancer antigen peptides as used herein encompass such derivatives.

Preferred length of the amino acid sequence of the cancer antigen peptide or its derivative is 8 to 15 amino acid residues, and more preferably 8 to 11 amino acid residues.
The cancer antigen peptide used in the invention may be any cancer antigen peptide which is already identified or will be identified in future. Preferably, considering the origin, HLA-type, and sex of the patient to be treated, the type of cancer to be treated and the like, the cancer antigen peptide which is expected to have high possibility to be recognized by the immune system of the patient may be selected and prepared.
In the invention, "set of cancer antigen peptides comprising two or more cancer antigen peptides" preferably comprises four or more, such as 14, cancer antigen peptide as described above.

In the method of the invention, it is first determined whether or not the patient has a peptide-specific cytotoxic T lymphocyte precursor which is specific to each peptide in the set of cancer antigen peptides. The determination may be conducted by a method known in the art, and for example by the method described in Example " 1.4 Cellular immune response" of the specification.
Then, the cancer antigen peptide to which the patient does not have a peptide-specific cytotoxic T lymphocyte precursor is removed from the set of cancer antigen peptides to provide an administration set of cancer antigen peptides. In the method of the present invention, this administration set of cancer antigen peptides is administered to the patient. The administration set of cancer antigen peptides may comprises one or more cancer antigen peptides, and preferably comprises two or more, more preferably 3 or 4 cancer antigen peptides. Although the number of cancer antigen peptides comprised in the administration set of cancer antigen peptides is not limited, it is preferably up to 4 considering the burden imposed on the patient.

The administration set of cancer antigen peptides can be prepared as a pharmaceutical composition and the composition can contain an adjuvant known to be used in vaccination. Alternatively, a pharmaceutical composition and an adjuvant which are prepared separately can be administered simultaneously.
The administration set of cancer antigen peptides can be administered subcutaneously, intradermally, or intravenously. Each peptide in the administration set of cancer antigen peptides may be administered separately or together. Specifically each peptide may be injected at independent sites, or a combination of all peptides may be injected at one site. Although the amount of the cancer antigen peptide in the vaccination may be adjusted as appropriate depending on, for example, the condition of disease to be treated, age and body weight of the particular patient, it is usually preferred to administer each peptide in the set in an amount of 0.1 mg to 10.0 mg, preferably 0.5 mg to 5.0 mg, more preferably 1.0 mg to 3.0 mg weekly or biweekly.

In the second aspect, the present invention provides a kit for conducting the method of the present invention, and specifically a kit for treating a cancer patient, which comprises a set of cancer antigen peptides comprising two or more cancer antigen peptides, and a reagent for determining whether or not the cancer patient has a peptide-specific cytotoxic T lymphocyte precursor which is specific to a cancer antigen peptide.
The set of cancer antigen peptides comprised in the kit is preferably lyophilized. The sets of cancer antigen peptides for determination and administration may be provided together, or separately as the first set for determination and the second set for administration.
The first set of cancer antigen peptides for determination is, for example, dissolved in a suitable solvent such as dimethylsulfoxide for use. After the dissolution in the solvent, e.g. in dimethylsulfoxide, it is stored in a low temperature, such as 4 °C, until use, if necessary.
The second set of cancer antigen peptides for administration may be dissolved or suspended in a physiologically acceptable solvent, such as saline. Alternatively, sodium bicarbonate can be used as a dissolving aid. The peptide solution can be further mixed with an adjuvant to form an emulsion before administeration.
Thus, the kit of the invention can comprise a solvent for the determination, a solvent for the administration, and an adjuvant. In addition, a part of the kit comprising the first set of cancer antigen peptides for determination and a reagent for the determination, and another part comprising the second set of cancer antigen peptides for administration and, if necessary, a solvent for administration and an adjuvant may be provided separately.

In the present invention, "reagent for determining whether or not a cancer patient has a peptide-specific cytotoxic T lymphocyte precursor which is specific to a cancer antigen peptide" comprises any material necessary for such determination by any method known in the art, for example, cells, cell culture medium, and plates used for determination which are necessary for such determination.

### EXAMPLES

The present invention is further illustrated by the following examples, but is not limited by them in any respect. Preparatory for the examples, 1. Eligibility criteria for patients, and 2. Patient characteristics are illustrated.

### 1. Eligibility criteria for patients

The Institutional Ethical Review Boards of Kurume University, Hokkaido University, Kochi University, and Kyoundo Hospital approved these clinical protocols of pre-designated peptide vaccination. Complete written informed consent was obtained from all of the patients at the time of enrollment. According to the protocol, patients were required to be positive for HLA-A24 or -A2 as determined by conventional serological typing of HLA-class I expression on PBMCs as reported previously (11,12). All patients were pathologically confirmed to have gastric adenocarcinoma or cervical carcinoma. Eligibility criteria included an age of 80 years or less, serum creatinine of less than 1.4 mg/dl, bilirubin of less than 1.5 mg/dl, platelet count of 100,000/µl or more, hemoglobin of 8.0 g/dl or more, and total white blood cells of 3,000/µl or more. Hepatitis B surface antigen and Hepatitis C RNA were required to be negative. The patients were untreated for at least 4 weeks before entry to the study, and had to have an Eastern Cooperative Oncology Group performance status of 0 to 2 at the time of entry. Patients with evidence of serious illness, immunosuppression, or autoimmune disease were excluded. Treatment was carried out from August 2001 through June 2003.

### 2. Patient characteristics

Eighteen patients (10 with recurrent uterine cervical cancer and 8 with advanced gastric cancer (GC)) were enrolled in this study. Detailed characteristics of these patients are shown in Table 1 hereinafter. The median age of the patients was 50, ranging from 27 to 74. Ten and 8 patients were HLA-A24 and -A2 positive, respectively. The sites of recurrence of cervical cancers were the vaginal stump (n = 4), lymph nodes (n = 4), lung (n = 1), and vulva skin (n = 1). The histology of cervical cancers was squamous cell carcinoma (n = 8) or adenocarcinoma (n = 2). Preceding treatments included surgical resection (n = 7), chemotherapy (n = 6), and radiotherapy (n = 10). Five, 2, and 1 GC cases were stages IV, IIIB, and IIIA based on the Japanese Classification of GC 13^{th} edition, respectively. The histology of GC was all adenocarcinoma with 6 cases (GC-3 to -8) being scirrhous (diffuse) types and the remaining 2 cases being non-scirrhous types. All GC patients underwent surgical resection of the primary lesion, and 6 of them had previously undergone failed chemotherapy.

### Table 1

**Table 1 Patient's characteristics**

| Case^{a} | Age/Sex | HLA type | PS^{b} | TNM (Stege)^{c} | Histology^{d} | Site of metastases^{e} | Previous treatments | | | No. of vaccination |
|---|---|---|---|---|---|---|---|---|---|---|
| | | | | | | | Surgery^{f} | Chemotherapy^{g} | Radiotherapy | |
| C-1 | 34/F | A24 | 0 | T1b1N0M0(1A) reccurent case | adeno | vaginal stump | RH | CDDP/CPT11 | whole pelvis | 13 |
| C-2 | 46/F | A24 | 0 | T4aNIMI (IVA) reccurent case | adeno | vaginal stump | TH | CDDP/CPT11 | whole pelvis | 15 |
| C-3 | 54/F F | A24 | 0 | T2bN1M0(IIB) reccurent case | SCC | paraortic LNs, Virchow LNs, lung | RH | CDDP / PEP | small pelvis | 2 |
| C-4 | 74/F F | A24 | 0 | T3bN0M0 (IIIB) reccurent case | SCC | vaginal stump | - | - | whole pelvis | 12 |
| C-5 | 52/F | A24 | I | T2bN1M0(IIB) reccurent case | SCC | lung | RH | - | whole pelvis | 12 |
| C-6 | 59/F | A2 | 0 | T1b1N1M0(IB) reccurent case | SCC | paraortic LNs | RH | - | whole pelvis | 12 |
| C-7 | 48/F | A2 | 0 | T3bN1M0(IIIB) reccurent case | SCC | vulva skin | - | BOMP | whole pelvis | 13 |
| C-8 | 27/F | A2 | 1 | T2bN1M0(IIB) reccurent case | SCC | vaginal stump | RH | CDDP / CPT 11 Taxol / CBDCA | small pelvis | 11 |
| C-9 | 50/F | A2 | 0 | T3bN1M0(IIIB) reccurent case | SCC | paraortic LNs, bone | - | CDDP | whole pelvis | 14 |
| C-10 | 59/F F | A2 | 0 | T2bN1M0 (IIB) reccurent case | SCC | pelvic LNs | RH | - | whole pelvis | 19 |
| GC-1 | 74/M | A24 | 0 | T3N2H0P0M0 (IIIB) reccurent case | adeno | abdominal lymphnodes | DG | 5FU, CDDP, 5'DFUR UFT, TS1, TXN | - | 15 |
| OC-2 | 68/M | A24 | 2 | T3NxH0P1M0(IV) | adeno | carcimomatosis peritonitis | TG+S | FP, TS-1, UFT | - | 8 |
| GC-3 | 28/F | A24 | 1 | T3NxHIPIM0(IV) | adeno | abdominal dessemination, liver, para-sortie lymphnodes | BSO | TS-1 | - | 7 |
| GC-4 | 50/F F | A24 | 1 | T3NxPIH0M0 (IV) | adeno | abdominal dessemination | TG | - | - | 9 |
| GC-5 | 74/F | A24 | 1 | 13NxH1P1M1 (IV) | adeno | abdominal lymphnodes, lung | TG | - | - | 3 |
| GC-6 | 56/M | A2 | 1 | T3N1H0P1M0(IV) | adeno | abdominal dessemination, carcinomatosis pleuritis | TG | TS-1 | - | 8 |
| GC.7 | 45/M | A2 | 2 | T3N2P0H0M0 (IIIB) reccurent case | adeno | abdominal dessemination, para-aortic lymphnodes | TG+ S | low dose FP MTX + LV | - | 4 |
| GC-8 | 50/M | A2 | 0 | T3NIH0P0M0 (IIIA) | | adeno abdominal dessemination | TG | 5FU. TS-1, CDDP | - | 6 |

| | | | | | | | | | | |
|---|---|---|---|---|---|---|---|---|---|---|
| ^{a}C; cervical cancer / GC; gastric cancer ^{b}Performance Status by ECOG score ^{c}Japanese Classification of Gastric Carcinoma the 13^{th} edition, and, UICC and FIGO Classification of Cervical Cancer (1997) ^{d}SCC, squamous cell carcinoma; adeno, adenocarcinoma ^{e}LNs, lymph nodes ^{f}RH, radical hysterectomy TH, total hysterectomy; DG. distal gastrectomy; TG, total gastrectomy; BSO, bilateral salphingo oophorectomy ^{g}CDDP, cisplatin; CPT11, irinotecan hydrochloride; BOMP, bleomycin hydrochloride + vincristine sulfate + mitomycinC + cisplatin; CBDCA, carboplatin; TSI, 1M tegafur-0.4M 5-chloro2,4-dihydroxyridine- M potassium oxonato; 5*DFUR, doxifluridine; 5FU, fluorouracil; LV, calcium leucovorin; MTX, methotraxate | | | | | | | | | | |

### Example 1

### Pre-designated vaccination

### 1.1 Peptides

The peptides used in the present study were prepared under the condition of Good Manufacturing Practice (GMP) by Multiple Peptide Systems (San Diego, CA). The sequences of the peptides are as follows: Peptides used for HLA-A24⁺ patients were SART3 109 (VYDYNCHVDL; SEQ ID No. 1), SART3 ₃₁₅ (AYIDFEMKI; SEQ ID No. 2), lck ₂₀₈ (HYTNASDGL; SEQ ID No. 3), lck ₄₈₈ (DYLRSVLEDF; SEQ ID No. 4), and SART2 ₉₃ (DYSARWNEI; SEQ ID No. 5). Those used for HLA-A2⁺ patients were CypB ₁₇₂ (VLEGMEVV; SEQ ID No. 6), lck ₂₄₆ (KLVERLGAA; SEQ ID No. 7), ppMAPkkk ₄₃₂ (DLLSHAFFA; SEQ ID No. 8), and UBE2V ₄₃ (RLQEWCSVI; SEQ ID No. 9). All of these peptides have demonstrated their ability to induce HLA-A24- or HLA-A2-restricted and tumor-specific CTL activity in the PBMCs of cancer patients (2-12). They were frequently administered to those cancer patients in the preceding personalized vaccination regimen in which cellular responses to peptides had been confirmed prior to vaccination (8-12). Although all peptides for HLA-A2⁺ patients were selected based on the binding motif to HLA-A*0201 molecules, these peptides are immunogenic not only in HLA-A*0201 patients but also in those with other HLA-A2 subtypes such as HLA-A*0206 or HLA-A*0207 (2, 11, 12).

### 1.2 Vaccination of the peptides

Each peptide was dissolved in a small amount of dimethylsulfoxide, and diluted with saline at a concentration of 4 mg/ml. Montanide ISA-51, an incomplete Freund's adjuvant, was manufactured by Seppeic, Inc (Franklin Lakes, NJ). One ml each of the resulting four different peptide emulsions (2 mg/peptide/injection) was injected subcutaneously in independent sites of the lateral abdominal wall for gastric cancer (GC) patients, while it was injected subcutaneously in independent sites of the lateral thigh for cervical cancer patients using a glass syringe as reported previously (11, 12). The interval of vaccination was 1 week and a total of 6 injections were performed.

### 1.3 Adverse events

All 18 patients were evaluated for adverse events. The vaccinations were generally well tolerated. Grade I and II fevers with mild flu-like symptoms were observed in 4 patients and 1 patient, respectively; these fevers were transient and self-limiting. Grade I and II local redness and swelling at the injection sites were observed in 12 and 4 patients, respectively. Nausea, vomiting, abdominal pain, and diarrhea were each observed in 2 patients, respectively. No severe (grade III or IV) toxicity was associated with the peptide vaccination. Grade I hematuria by bladder invasion and vaginal bleeding by vaginal stump were each observed in one case, while grade IV of vomiting of blood caused by tumor progression was observed in one GC patient (GC-7). There was no clinical evidence of an autoimmune reaction as determined by symptoms, physical examination, or laboratory tests.

### 1.4 Cellular immune responses

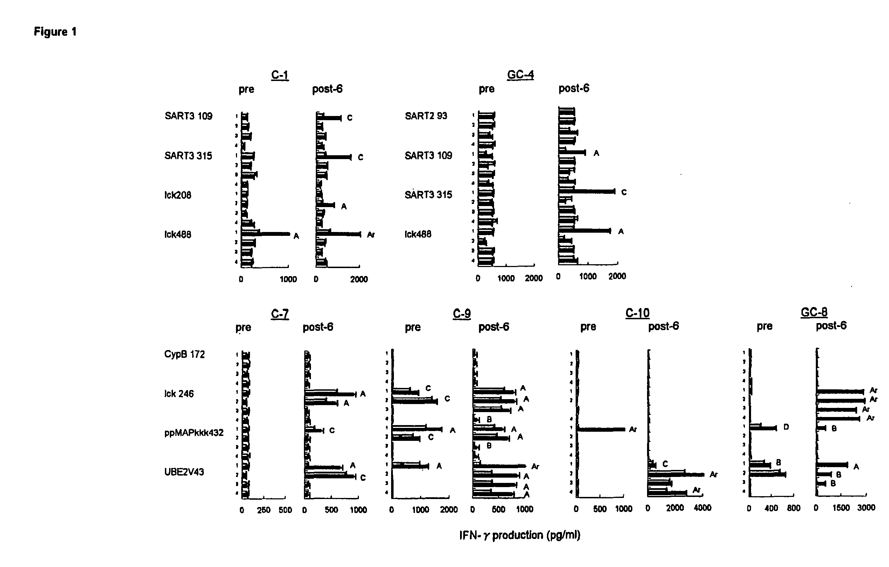

For measurement of cellular responses, PBMCs were separated by means of Ficoll-Conray density gradient centrifugation then used for CTL precursors assay as reported previously (11, 12). Evaluation of cellular immune responses to the administered peptides were provided in Table 2. To evaluate the cellular immune responses to the vaccinated peptides, pre- and post (6^{th})-vaccination PBMCs (1 x 10⁵ cells/well) were incubated with 10 µM of each peptide in four different wells of a 96-well microculture plate (Nunc, Roskilde, Denmark) in 200 µl culture medium containing interleukin (IL)-2 (100 units/ml), as reported previously (12). On culture day 14, the peptide-stimulated PBMCs (8-12 x 10⁵ /well) from each well were independently collected and divided into the four equal portions. Two such portions were separately tested for their ability to produce interferon (IFN)-y in response to C1R-A2402 cells (Oiso M, Eura M, Katsura F, Takiguchi M, Sobao Y, Masuyama K, Nakashima M, Itoh K, Ishikawa T. A newly identified MAGE-3-derived epitope recognized by HLA-A24-restricted cytotoxic T lymphocytes. Int. J. Cancer 81: 387-394, 1999) for HLA-A24⁺ or T2 cells (HLA-A*0201-expressing cell line, ATCC: CRL-1992) for HLA-A2⁺ PBMCs in the duplicate assays, while the remaining two portions were tested for a negative control peptide (HIV peptide, RYLRDQQLL for HLA-A24 and SLYNTVATL for HLA-A2) as reported previously (10-12). Background IFN-γ production in response to the HIV peptide (<50 pg/ml) was subtracted. After an 18 hr incubation, the supernatant was collected for measurement of IFN-γ by enzyme-linked immunosorbent assay (ELISA) (limit of sensitivity: 10 pg/ml). A two-tailed Student's t-test was employed for statistical analysis. Four representative cases of cervical cancer (C-1, 7, 9, and 10) and 2 GC cases (GC-4 and 8) are shown in Figure 1. A detailed summary is provided in Table 2. Criteria for evaluation of cellular responses were also described in the legend of Table 2.

### Table 2

**Table 2 Summary of responses to the peptide vaccination**

| case | Peptide | Peptide-specific CTLp^{a} | | Antibody to peptide^{b} | | DTH^{c} (Post) | Clinical response after^{d} | | TTP^{a} (days) | OS^{f} (months) |
|---|---|---|---|---|---|---|---|---|---|---|
| | | Pre | Post 6th | Pre | Post 6th | | 6th | 12th | | |
| C-1 | SART3 109 | - | C | - | + | - | SD | PD | 152 | 10+ |
| | SART3 315 | - | C | - | - | +(3) | | | | |
| | lck 208 | | A | | | | | | | |
| | lck 488 | A | Ar | - | - | +(4) | | | | |
| C-2 | SART3 109 | D | - | - | - | +(5) | SD | PD | 126 | 9+ |
| | SART3 315 | - | A | - | - | | | | | |
| | | | | | | | | | | |
| | lck 488 | - | CC | - | - | - | | | | |
| C-3 | SART3 109 | - | NT | - | NT | - | dead | - | 20 | 1 |
| | SART3 315 | - | NT | - | NT | - | | | | |
| | lck 208 | - | NT | - | NT | - | | | | |
| | lck 488 | - | NT | - | NT | - | | | | |
| C-4 | SART3 109 | C | - | + | + | - | SD | SD | - | 5+ |
| | SART3 315 | - | - | - | + | - | | | | |
| | lck 208 | - | - | - | + | - | | | | |
| | lck 488 | A | - | - | - | - | | | | |
| C-5 | SART3 109 | - | - | - | - | - | PD | dead | 41 | 5 |
| | SART3 315 | - | - | - | - | - | | | | |
| | lck 208 | - | - | - | - | - | | | | |
| | lck 488 | AC | B | - | - | - | | | | |
| C-6 | CypB172 | - | - | - | + | - | PD | PD | 43 | 15+ |
| | lck 246 | - | A | - | + | - | | | | |
| | ppMAPkkk 432 | - | CCCC | - | - | - | | | | |
| | UBE2V 43 | Ar | AA | - | + | +(4) | | | | |
| C-7 | CypB 172 | - | - | - | | - | PD | dead | 65 | 8 |
| | lck 246 | - | AA | - | + | - | | | | |
| | ppMAPkkk: 432 | - | C | - | - | - | | | | |
| | UBE2V 43 | - | AC | - | + | +(3) | | | | |
| C-8 | CypB 172 | - | A | - | - | - | PD | dead | 39 | 6 |
| | lck 246 | A | - | - | - | - | | | | |
| | ppMAPkkk 432 | - | - | - | - | - | | | | |
| | UBE2V 43 | - | - | - | + | - | | | | |
| C-9 | CypB 172 | - | - | - | - | - | SD | SD | - | 7+ |
| | lck 246 | CC | AAAB | - | - | - | | | | |
| | ppMAPkkk 432 | AC | AAB | - | - | - | | | | |
| | UBE2V 43 | A | ArAAA | - | - | - | | | | |
| C-10 | CypB 172 | - | - | - | + | - | SD | PD | 135 | 12+ |
| | lck 246 | - | - | - | + | - | | | | |
| | ppMAPkkk 432 | Ar | - | - | - | - | | | | |
| | UBE2V 43 | - | ArArC | - | + | - | | | | |
| GC-1 | SART2 93 | NT⁵ | ArC | - | - | - | SD | PD | 89 | 5 |
| | SART3 109 | NT | AA | + | + | - | | | | |
| | SART3 315 | NT | A | - | - | - | | | | |
| | lck 488 | NT | A | - | - | - | | | | |
| GC-2 | SART2 93 | - | - | - | - | - | SD | dead | 53 | 2 |
| | SAR73 109 | - | - | - | - | - | | | | |
| | SART3 315 | - | - | - | - | - | | | | |
| | lck 488 | A | Ar | - | - | - | | | | |
| GC-3 | SART2 93 | NT | - | - | - | - | dead | | 43 | 2 |
| | SART3 109 | NT | - | - | - | - | | | | |
| | SART3 315 | NT | C | - | - | - | | | | |
| | lck 488 | NT | - | - | - | - | | | | |
| GC-4 | SART2 93 | - | - | - | - | - | PD | dead | 27 | 2 |
| | SAR73 109 | - | A | + | + | - | | | | |
| | SART3 315 | - | C | - | - | - | | | | |
| | lck 488 | - | A | - | - | - | | | | |
| GC-5 | SART2 93 | A | NT | - | NT | - | dead | - | 14 | 1 |
| | SART3 109 | - | NT | - | NT | - | | | | |
| | SART3 315 | - | NT | - | NT | - | | | | |
| | lck 488 | - | NT | - | NT | - | | | | |
| GC-6 | CypB 172 | - | B | - | - | | PD | dead | 47 | 2 |
| | lck 246 | - | AB | + | + | - | | | | |
| | ppMAPkkk 432 | - | B | - | - | - | | | | |
| | UBE2V 43 | - | - | + | + | - | | | | |
| GC-7 | CypB 172 | - | NT | - | NT | - | dead | - | 17 | 1 |
| | lck 246 | - | NT | - | NT | - | | | | |
| | ppMAPkkk 432 | C | NT | - | NT | - | | | | |
| | UBE2V 43 | - | NT | + | NT | - | | | | |
| GC-8 | CypB 172 | - | - | - | - | - | SD | SD | - | 11+ |
| | lck 246 | D | ArArArAr | - | - | - | | | | |
| | ppMAPkkk 432 | D | B | - | - | - | | | | |
| | UBE2V 43 | BD | ABB | - | - | - | | | | |

| | | | | | | | | | | |
|---|---|---|---|---|---|---|---|---|---|---|
| ^{a} The CTL precursor induction assay was performed in quadruplicate, and the background response to the HIV peptide was substracted from the value. The result was evaluated by the following criteria: Ar (p value ≤0.1 and IFN-γ production ≥500 pg/ml), A (p≤0.05 and IFN-γ production >50), B (p<0.05 and 25 <IFN-γ production <50), C (0.05<p<0.1 and IFN-γ production >50), and D (0.05 <p< 0. 1 and 25 <IFN-γ production <50). The classification is shown by letters of the alphabet, and each character represents the results of each well. For example, AAB means that three wells were judged as A, A, and B, and one well was negative. ^{b} The net OD value of >0.02 (OD value in response to a corresponding peptide subtracted from that in response to an HIV peptide at a serum dilution of 100) was considered as positive of peptide-reactive IgG. Number of the vaccination when DTH to the peptide was detected for the first time. ^{c} SD, stable disease, PD, progressive disease. TTP, time to progression. OS, overall survival. Pulse (+) mark; Patients are alive (2003.9.15). NT, not tested. | | | | | | | | | | |

Both the pre- and post (6^{th})-vaccination PBMCs were thawed at the same time in order to avoid any in vitro biases during the assay of peptide-specific CTL precursors. The pre-vaccination PBMCs from the two cases (GC-1 and GC-3) were not available for analysis because of the limited numbers of cells, and the post-vaccination PBMCs from three cases (C-3, GC-5, and GC-7) were not available because of rapid progression and death. The assay of peptide-specific CTL precursors was performed in quadruplicate, and the amount of IFN-γ in response to an HIV peptide was subtracted. The results were assessed in terms of two parameters, the P value and IFN-γ production, as described in the table legend. In total, peptide-specific CTL precursors were induced in 9 of 13 patients (69%). For example, in one HLA-A24⁺ GC patient (GC-4), none of the pre-vaccination PBMCs responded to any of the four vaccinated peptides, while each single well of the post-vaccination PBMCs responded to the SART3₁₀₉ peptide, the SART3₃₁₅ peptide, or the lck₄₈₈ peptide. In an HLA-A2⁺ cervical cancer patient (C-9), one well of the pre-vaccination PBMCs responded to the UBE2V₄₃ peptide, 2 wells responded to the lck₂₄₆ peptide, and 2 wells responded to the ppMAPkkk₄₃₂ peptide. On the other hand, 4, 4, and 3 wells of the post-vaccination PBMCs responded to the UBE2V₄₃ peptide, the lck₂₄₆ peptide, and the ppMAPkkk₄₃₂ peptide, respectively. These results indicate that the pre-designated regimen has the potential to effectively induce peptide-specific CTLs.

### 1.5 Humoral immune responses

We recently reported that the induction of IgGs reactive to administered peptides correlates well with the clinical responses and outcomes of patients with several types of cancer (8, 10, 11). Therefore, we examined the levels of peptide-specific IgGs kinetically. In brief, 100 µl/well of serum samples from pre- and post (6^{th})-vaccinations were diluted with 0.05% Tween 20-Block Ace, and were added to the peptide (20 µg/well)-immobilized plate. After a 2 hr incubation at 37 °C, the plate was washed and further incubated for another 2 hr with a 1:1000-dilluted rabbit anti-human IgG (γ-chain-specific, DAKO, Glostrup, Denmark). The plate was washed, then 100 µl of 1: 100-diluted goat anti-rabbit IgG-conjugated horseradish peroxidase-dextran polymer (EnVision, DAKO) was added to each well, and the plate was incubated for 40 min. After washing, 100 µl/well of tetramethylbenzidine substrate solution (KPL, Guildford, UK) was added, and the reaction was stopped by an addition of 1 M phosphoric acid. The optical density (OD) values of each sample were shown as OD units/ml. For determining the cut-off value, patients' sera were measured at the same time for their reactivity to both corresponding peptides and an HN peptide, taken as a negative control peptide. OD value (calculated by subtracting the OD value in response to an HN peptide at a serum dilution of 1: 100 from that in response to a corresponding peptide) of >0.02 was considered as positive of peptide-reactive IgG.

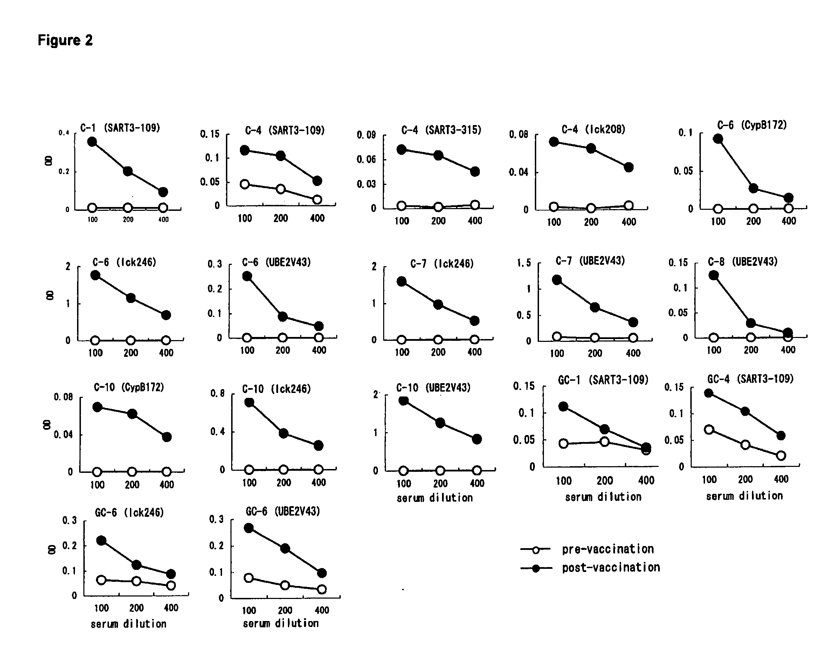

As shown in Table 2, IgGs reactive to the peptides were detected in the pre-vaccination, and peptide-specific IgGs increased after the peptide vaccination (C-4, GC-1, 4, and 6). In addition, IgGs reactive to the administered peptides were induced in post-vaccination sera from 6 of 15 patients tested (C-1, 4, 6, 7, 8, and 10). In total, vaccine-induced induction or augmentation of peptide-specific IgGs was observed in the post-vaccination sera of 9 of 15 patients tested (60%). The results of 9 cases whose sera had significant levels of IgG reactive to at least one peptide are shown in Figure 2. In one HLA-A2⁺ GC patient (GC-6), significant levels (0.02< OD value at a serum dilution of 1:100) of IgG specific to the UBE2V₄₃ and lck₂₄₆ peptides, but not to the other 2 peptides, were detectable in the pre-vaccination sera of this patient, and the peptide vaccination increased the level of peptide-specific IgG. The peptide specificity of each of these IgGs was shown elsewhere (6-12).

### 1.6 Delayed-type hypersensitivity (DTH) reaction

Skin tests were performed by intradermal injection of 50 µg of each peptide using a tuberculin syringe with a 26-gauge needle. Saline was used as a negative control. Immediate- and delayed-type hypersensitivity (DTH) reactions were determined at 20 min and 24 h after the injection, respectively. At least 10 mm of induration or 20 mm of erythema read 24 h after injection was needed to score the DTH skin test as positive.
No delayed-type hypersensitivity (DTH) reaction to the peptides was observed before the vaccination in any of the patients tested. Peptide-specific DTH reactions were induced in 4 cervical cancer patients (C-1, 2, 6, and 7) until the 6^{th} vaccination; the details of these reactions are provided in Table 2. For example, in patient C-1, DTH reactions to Ick₂₀₈ and lck₄₈₈ peptides were observed after the 3^{rd} and 4^{th} vaccinations, respectively.

### 1.7 Evaluation of clinical response

All known sites of disease were evaluated by CT-scan or X-ray examination before and after every 3 to 6 vaccinations. Patients were assigned to a response category according to the response evaluation criteria for solid tumors, a revised version of the WHO criteria published in June 1999 in the WHO Handbook for reporting results of cancer treatment.

The clinical responses are summarized in Table 2. Major tumor regression (> 50% reduction) was not observed in any of the patients. Four patients (C-3, GC-3, -5, and -7) had rapid progression and died due to cancer before the 6^{th} vaccination. At the time of the 6^{th} vaccination, among the 14 patients eligible for evaluation, 8 cases were diagnosed with stable disease (SD) and the remaining 6 patients were diagnosed with progressive disease (PD). All of these 14 patients decided for themselves to enter into the personalized vaccination regime based on the screening of CTL precursors to peptides with the post (6^{th})-vaccination PBMCs. At the time of the 12^{th} vaccination, among the 8 patients eligible for evaluation, 3 cases (C-4, -9, and GC-8) were diagnosed with SD, and the remaining 5 patients were diagnosed with PD. Tumor regression by means of a 16% size reduction of a paraortic lymph node was observed at the 10^{th} vaccination in one case (C-9). The median observation time, median time to progression (TTP), and median survival time (MST) of 18 patients were 11, 1.5 (47 days), and 5 months (156 days), respectively.

Analysis of the 3 SD patients may be useful for the evaluation of prognostic factors. In patient C-4, although peptide-specific IgGs was induced, peptide-specific CTLs and DTH were not observed after the peptide vaccination. In patients C-9 and CG-8, although peptide-specific CTLs were induced, peptide-specific IgGs and DTH were not observed. In comparison to the other 15 patients, one common finding among these 3 SD patients was that peptide-specific CTL precursors to more than 2 peptides were detected in the pre-vaccination PBMCs. Namely, CTL precursors to 3 of 4 vaccinated peptides were detectable in the pre-vaccination PBMCs of patient C-9, and CTL precursors to 2 of 4 vaccinated peptides were detectable in the pre-vaccination PBMCs of patients C-4 and GC-8. The results are summarized in Table 3.

**Table 3 Prospective study (cervical cancer and gastric cancer) Correlation between the number of pre-vaccination peptide-specific CTL precursors and clinical responses in the pre-designated regimen**

| Number of pre-vaccination peptide-specific CTL precursors | Cases | Clinical responses (Time to progression) |
|---|---|---|
| 4 | 0 | |
| 3 | 1 | MR (C-9: 7M+) |
| 2 | | SD (C-4: 5M+, GC-8: 11 M+) |
| 1 | 9 | All PD (GC-9: <1 M, GC-7: <1 M, C-5: 1 M, C-6: 1M, C-8: 1M, GC-2: 2M, C-2: 4M, C-10: 4M, C-1: 5M) 4 alive |
| 0 | 4 | All PD (C-3: <1 M, GC-4: <1 M, GC-6: <2M, C-7: 2M) All dead |

| | | |
|---|---|---|
| MR, minor response; SD, stable disease; PD, progressive disease | | |

### 1.8 Shorter survival of patients in the pre-designated vaccination regimen

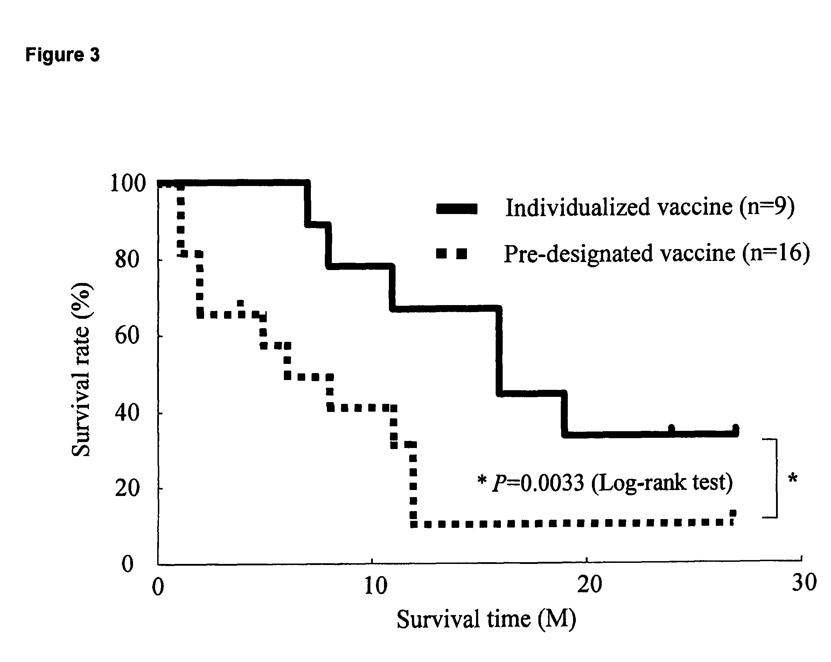

In this study, we carried out a new trial of the"pre-designated" regimen, in which cancer patients were vaccinated with peptides that were frequently selected as vaccine candidates in the preceding personalized regimen. However, the clinical response seemed to be inferior to that of the personalized regimen. Therefore, we compared the overall survival time of cervical and scirrhous GC patients under this pre-designated regimen (n = 16) to that under the preceding personalized vaccination regimen (n = 9, 5 with cervical cancer and 4 with scirrhous GC) (Figure 3). As a result, the overall survival of the patients in this regimen was significantly (p=0.0033 by Log-rank test) shorter than that of the preceding personalized regimen.

### 1.9 Incidental suppression of pre-existing immune responses by the pre-designated vaccination

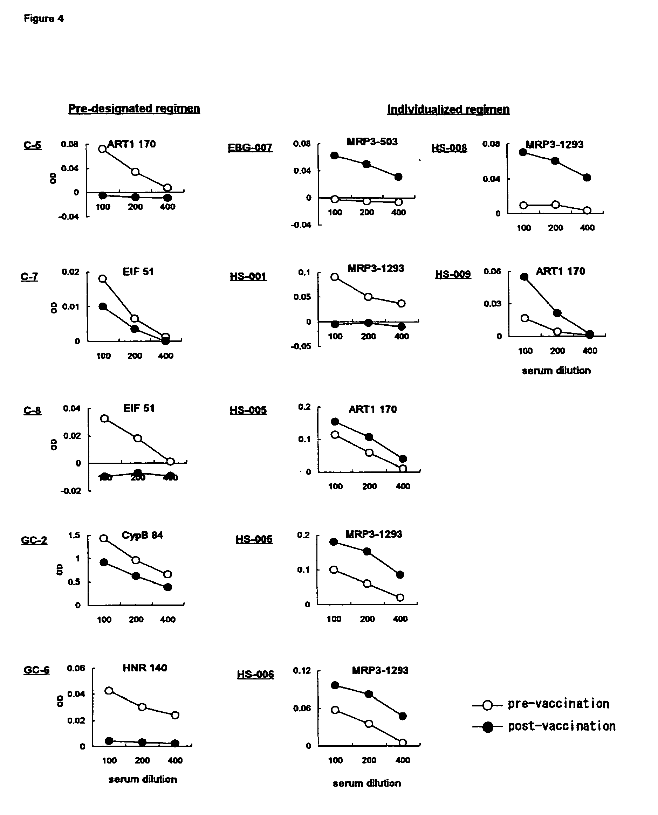

As for the mechanism of the shorter survival of the patients in the pre-designated regimen, it is possible that, in the pre-designated regimen, pre-existing immune responses were incidentally suppressed by the induction of primary immune responses to the administered peptides for which CTL precursors did not exist. To test this possibility, we compared the levels of IgG reactive to vaccinated peptides with that reactive to non-vaccinated peptides. The pre- and post (6^{th})-vaccination sera from 15 patients were tested for their levels of IgG specific to 6 different HLA-A24-restricted CTL epitope peptides (CypB₈₄, ART1₁₇₀, ART4₁₃, ART4₇₅, MRP3₅₀₃, and MRP3₁₂₉₃) in HLA-A24⁺ cases (n = 8), and to 5 different HLA-A2-restricted CTL epitope peptides (WHSC₁₀₃, WHSC₁₄₁, HNRPL₁₄₀, HNRPL₅₀₁, and EIF₅₁) in HLA-A2⁺ cases (n = 7). None of the peptides were administered as vaccines in the pre-designated regimen, but all of them were administered in the preceding personalized regimen, and the peptide-specificity of serum IgG was reported elsewhere (8-14). The results are shown in Figure 4. The levels of peptide-specific IgGs in the sera from 5 of 15 cases (C-5, 7, 8, GC-2, and 6) decreased after the pre-designated vaccination as shown in the left column of Figure 4. It is noteworthy that the TTPs of these 5 patients were very short (39, 41, 47, 53, and 65 days), and the median TTP was 43 days. Those from the personalized vaccination regimen were also provided for their reactivity to the non-vaccinated peptides, and the results from 6 cases whose post-vaccination sera showed increased or decreased responses to the specific peptides are shown in the right column of Figure 4. Detailed results for the personalized vaccinations were described elsewhere (11, 12). In the personalized regimen, peptide-specific IgG increased after the vaccination with the exception of patient HS-001 with the MRP3-1293 peptide.

### 1.10 Discussion

There are no apparent differences between the two regimens with regard to patient characteristics, except for the numbers of cervical cancer patients (10 in the pre-designated regimen vs 5 in the preceding personalized regimen). Increased cellular responses to some of the vaccinated peptides were observed in the post-vaccination PBMCs of 11 of 13 patients (85) and in 26 of the 52 peptides (50) tested in this regimen, while they were observed in 11 of 18 patients (61) and in 9 of the 48 peptides (18.8) tested in the preceding personalized regimen (11, 12). Humoral responses to peptides were observed in the post-vaccination sera of 9 of 15 patients tested (60) and in 17 of the 60 peptides (28.3) in the pre-designated regimen, while they were observed in 13 of 15 patients (87) and in 20 of the 56 peptides (35.7) in the preceding personalized regimen. These results indicate that the pre-designated regimen is superior to the personalized regimen with regard to the induction of the cellular responses to the administered peptides, whereas in the case of humoral responses the personalized regimen seems to be superior. However, a major difference in clinical response was observed between these regimens. Namely, the median TTP (n = 16, 1.5 months) of patients with cervical cancer (n = 10, 2 months) and scirrhous GC (n = 6, 1 month) under this regimen was much shorter than that (n = 9, 15 months) of patients with cervical cancer (n = 5, 15 months) and scirrhous GC (n = 4, 7 months) who underwent the preceding personalized regimen (11, 12), respectively. The overall survival of these patients under this pre-designated regimen was significantly shorter than that under the preceding personalized regimen (Figure 3).

The immunological mechanisms involved in the shorter survival associated with the pre-designated regimen are presently unclear. However, one possibility is that the induction of potent primary immune responses to the administrated peptides might suppress pre-existing specific immune responses to tumor cells. To test this possibility, we examined the levels of serum IgGs reactive to CTL-epitope peptides. These levels were often found in both pre-vaccinated cancer patients and healthy donors, as reported previously (8-12, 14, 15). As a result, the levels of peptide-specific IgG in the pre-vaccination sera from 5 of 15 cases were found to be lower than those in the post-vaccination sera in association with the shorter TTP in these patients. With regard to peptides, the IgGs reactive to 5 among 11 kinds of peptides were found to be decreased. In contrast, such decreased humoral responses to non-vaccinated peptides were rarely found in the post-vaccination sera of patients under the personalized regimens.

### REFERENCES

The following references are incorporated herein by reference.
1. Shichijo S, Nakao M, Imai Y, et al.: A gene encoding antigenic peptides of human squamous cell carcinoma recognized by cytotoxic T lymphocytes. J Exp Med 187: 277-288, 1998.
2. Ito M, Shichijo S, Tsuda N, et al.: Molecular basis of T cell-mediated recognition of pancreatic cancer cells. Cancer Res 61: 2038-2046, 2001.
3. Yang D, Nakao M, Shichijo S, et al.: Identification of a gene coding for a protein possessing shared tumor epitopes capable of inducing HLA-A24-restructed Cytotoxic T lymphocytes in cancer patients. Cancer Res 59: 4056-4063, 1999.
4. Yamada A, Kawano K, Koga M, Takamori S, Nakagawa M, Itoh K: Gene and peptide analyses of newly defined lung cancer rejection antigens recognized by HLA-A2402-restricted tumor-specific cytotoxic T lymphocytes. Cancer Res 63: 2829-2835, 2003.
5. Harashima N, Tanaka K, Sasatomi T, et al.: Recognition of the Lck tyrosine kinase as a tumor antigen by T cells of metastatic cancer patients. Eur J Immunolo 31: 323-332, 2001.
6. Gohara R, Imai N, Rikimaru T, et al.: Phase 1 clinical study of cyclophilin B peptide vaccine for lung cancer patients. J Immunother 25: 439-444, 2002.
7. Miyagi Y, Imai N, Sasatomi T, et al.: Induction of cellular immune responses to tumor cells and peptides in colorectal cancer patients by vaccination with SART3 peptides. Clin Cancer Res 7: 3950-3962, 2001.
8. Mine T, Gouhara R, Hida N, et al.: Immunological evaluation of CTL precursor-oriented vaccines for advanced lung cancer patients. Cancer Sci 94: 548-556, 2003.
9. Tanaka S, Harada M, Mine T, et al.: Peptide vaccination for patients with melanoma and other types of cancer based on pre-existing peptide-specific cytotoxic T lymphocyte precursors in the periphery. J Immunother 26: 357-366, 2003.
10. Noguchi M, Kobayashi K, Suetsugu N, et al.: Induction of cellular and humoral immune responses to tumor cells and peptides in HLA-A24 positive hormone-refractory prostate cancer patients by peptide vaccination. Prostate 57: 80-92, 2003.
11. Sato Y, Shomura H, Maeda Y, et al.: Immunological evaluation of peptide vaccination for patients with gastric cancer based on pre-existing cellular response to peptide. Cancer Sci 94: 802-808, 2003.
12. Tsuda N, Mochizuki K, Harada M, et al.: Vaccination with pre-designated or evidence-based peptides for patients with recurrent gynecologic cancers. J Immunother (in press), 2003.
13. Ohkouchi S, Yamada A, Imai N, et al.: Non-mutated tumor-rejection antigen peptides elicit type-I allergy in the majority of healthy individuals. Tissue Antigens 59: 259-272, 2002.
14. Kawamoto N, Yamada A, Ohkouchi S, et al.: IgG reactive to CTL-directed epitopes of self-antigens is enter lacking or unbalanced in atopic dermatitis patients. Tissue Antigens 61: 352-361, 2003.
15. Rosenberg SA, Yang JC, Schwartzentruber DJ, et al.: Immunologic and therapeutic evaluation of a synthetic peptide vaccine for the treatment of patients with metastatic melanoma. Nature Med 4: 321-327, 1998.
16. Rosenberg, SA,: A new era for cancer immunotherapy based on the genes that encode cancer antigens. Immunity 10: 281-287, 1999.
17. van Driel WJ, Ressing ME, Kenter GG, et al.: Vaccination with HPV 16 peptides of patients with advanced cervical carcinoma: clinical evaluation of a phase I-II trial. Eur J Cancer 35: 946-952, 1999.
18. Nestle FO, Alijagic S, Gilliet M, et al.: Vaccination of melanoma patients with peptide- or tumor lysate-pulsed dendritic cells. Nat Med 4: 328-332, 1998.
19. Gjertsen MK, Buanes T, Rosseland AR, et al.: Intradermal ras peptide vaccination with granulocyte-macrophage colony-stimulating factor as adjuvant: Clinical and immunological responses in patients with pancreatic adenocarcinoma. Int J Cancer 92: 441-450, 2001.
20. Lau R, Wang F, Jeffery G., et al.: Phase I trial of intravenous peptide-pulsed dendritic cells in patients with metastatic melanoma. J Immunother 24: 66-78, 2001.
21. Jager E, Gnjatic S, Nagata Y, et al.: Induction of primary NY-ESO-1 immunity: CD8+ T lymphocyte and antibody responses in peptide-vaccinated patients with NY-ESO-1+ cancers. Proc Natl Acad Sci USA 97: 12198-12203, 2000.

## Claims

1. A method for treating a cancer patient which comprises administering a therapeutically effective amount of a cancer antigen peptide to the patient in need thereof, wherein the method comprises the steps of
1) providing a set of cancer antigen peptides comprising two or more cancer antigen peptides,
2) determining whether or not the patient has a peptide-specific cytotoxic T lymphocyte precursor which is specific to each peptide in the set of cancer antigen peptides,
3) removing the cancer antigen peptide to which the patient does not have the peptide-specific cytotoxic T lymphocyte precursor from the set of cancer antigen peptides to provide an administration set of cancer antigen peptides,
4) administering the administration set of cancer antigen peptides to the patient.

2. The method of Claim 1, wherein the set of cancer antigen peptides comprises four or more cancer antigen peptides.

3. The method of Claim 1, wherein the administration set of cancer antigen peptides comprises three or four cancer antigen peptides.

4. A kit for treating a cancer patient, which comprises
a set of cancer antigen peptides comprising two or more cancer antigen peptides, and
a reagent for determining whether or not the cancer patient has a peptide-specific cytotoxic T lymphocyte precursor which is specific to a cancer antigen peptide.
